(19) Europäisches Patentamt
European Patent Office
Office européen des brevets

(11) Veröffentlichungsnummer: **0 321 817 B1**

(12) **EUROPÄISCHE PATENTSCHRIFT**

(45) Veröffentlichungstag der Patentschrift: **04.03.92**

(51) Int. Cl.⁵: **A61K 33/40**, A61K 31/19, A61K 31/155, //(A61K33/40, 31:19,31:155,31:045)

(21) Anmeldenummer: **88120705.4**

(22) Anmeldetag: **12.12.88**

(54) **Verwendung von Bisguanide enthaltenden Zusammensetzungen gegen Eier des Madenwurms.**

(30) Priorität: **21.12.87 DE 3743374**

(43) Veröffentlichungstag der Anmeldung:
**28.06.89 Patentblatt 89/26**

(45) Bekanntmachung des Hinweises auf die Patenterteilung:
**04.03.92 Patentblatt 92/10**

(84) Benannte Vertragsstaaten:
**AT BE CH DE ES FR GB GR IT LI LU NL SE**

(56) Entgegenhaltungen:
**EP-A- 0 024 031** **FR-A- 2 282 262**
**US-A- 2 990 425** **US-A- 3 468 898**
**US-A- 4 022 834** **US-A- 4 053 636**

(73) Patentinhaber: **Henkel Kommanditgesellschaft auf Aktien**
**Postfach 1100 Henkelstrasse 67**
**W-4000 Düsseldorf 1(DE)**

(72) Erfinder: **Guhl, Walter, Dr.**
**Wiederhoferstrasse 27**
**W-5657 Haan(DE)**
Erfinder: **Bansemir, Klaus, Dr.**
**Ursulaweg 51**
**W-4018 Langenfeld(DE)**

**EP 0 321 817 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von Bis- bzw. Oligobisguanide, Alkohole, Wasserstoffperoxid bzw. Wasserstoffperoxid freisetzenden Verbindungen, Carbonsäuren und Wasser enthaltenden Zusammensetzungen zur äußerlichen Anwendung gegen Eier des Madenwurms Enterobius vermicularis auf der Haut.

Weltweit sollen etwa 50 % aller Menschen Träger des Madenwurms Enterobius vermicularis (Oxyuris) sein. Diese Parasitose kommt häufig auch in Mitteleuropa vor und stellt insbesondere bei Kleinkindern ein Problem dar.

Die Verbreitung des Madenwurms kann auf zwei Arten erfolgen, wobei die infektiösen Stadien (embryonierte Eier) aerogen durch Luft- bzw. Schmierinfektionen aufgenommen (meist relativ schwache Primärinfektion) oder direkt durch Kratzen im Analbereich über die Hände in den Mund gebracht (massive Reinfektion) werden. Für die Reinfektion spielt der Vermehrungszyklus der Oxyuren eine wesentliche Rolle. Nach der Primärinfektion und erster Besiedelung im Darm legen die weiblichen Oxyuren während der Nachtruhe im Analbereich ihre Eier ab, welche entweder durch Händekontakt von der Analhaut zum Mund oder aerogen oral aufgenommen werden. Pro $cm^2$ infizierter Analhaut können bis zu 1200 Eier gefunden werden, die durch Waschen mit Seifenlösung häufig nicht vollständig entfernt werden können.

Darüber hinaus besteht dabei die Gefahr, daß über Waschlappen und Hände eine Verbreitung auf andere Familienmitglieder stattfindet.

Bis- bzw. Oligobisguanide die im Rahmen der Erfindung geeignet sind, sind als desinfizierend wirkende Verbindungen bekannt, vgl. GB-PS 702 286, 1 152 243; DE-OS 24 37 844, EP-OS 00 24 031, US-PS 2 284 924, 2 990 425, 3 468 898, 4 022 834, 4 053 636; DE-OS 22 12 259, 26 27 548. Einer der bekanntesten Vertreter dieser Verbindungen ist 1,6-Bis-($N^5$-p-chlorphenyl-$N^1$-biguanido)-hexan, das unter der INN-Bezeichnung Chlorhexidin (als Gluconat in wäßrig/alkoholischen Lösungen) in Desinfektionsmitteln verwendet wird. Zur Abtötung von Eiern der Oxyuren sind diese Desinfektionsmittel jedoch nicht geeignet.

Es wurde nun überraschend gefunden, daß die Verwendung von Zusammensetzungen der eingangs genannten Art bei äußerlicher Anwendung eine wirksame Bekämpfung des Madenwurms Enterobius vermicularis ermöglicht; dabei werden die Eier innerhalb von wenigen Minuten abgetötet. Zweckmäßigerweise sollte diese Behandlungsweise von einer internen Wurmtherapie mittels hierfür üblicher Wurmmittel begleitet werden.

Als Bis-bzw. Oligobisguanide eignen sich die in den vorstehend genannten Vorveröffentlichungen geeigneten Verbindungen, vorzugsweise diejenigen Verbindungen, die aus der von
1,2-Bis-($N^5$-p-chlorphenyl-$N^1$-biguanido)-ethan,
1,2-Bis-($N^5$-p-nitrophenyl-$N^1$-biguanido)-ethan,
1,2-Bis-($N^5$-p-hydroxyphenyl-$N^1$-biguanido)-ethan,
1,2-Bis-($N^5$-p-chlorbenzyl-$N^1$-biguanido)-ethan,
1,2-Bis-($N^5$-p-bromphenyl-$N^5$-hexyl-$N^1$-biguanido)-ethan,
1,2-Bis-($N^5$-p-chlorphenyl-$N^5$-2-ethylphenyl-$N^1$-biguanido)-ethan,
1,2-Bis-($N^5$-p-chlorphenyl-$N^1$-ethyl-$N^1$-biguanido)-ethan,
1,2-Bis-($N^5$-p-methoxyphenyl-$N^1$-biguanido)-ethan,
1,2-Bis-($N^5$-p-methylphenyl-$N^1$-biguanido)-ethan,
1,2-Bis-($N^5$-3,5-dimethylphenyl-$N^1$-biguanido)-ethan,
1,2-Bis-($N^5$-2,6-dichlorphenyl-$N^1$-biguanido)-ethan,
1,2-Bis-($N^5$-2,6-dimethylphenyl-$N^1$-biguanido)-ethan,
1,4-Bis-($N^5$-p-chlorphenyl-$N^1$-biguanido)-butan,
Bis-($N^5$-p-chlorphenyl-$N^1$-biguanido)-methan,
1,3-Bis-($N^5$-p-chlorphenyl-$N^1$-biguanido)-propan
1,6-Bis-($N^5$-p-chlorphenyl-$N^1$-biguanido-hexan
und Oligobisguaniden der allgemeinen Formel

$$\{(CH_2)_6\text{-NH-C(NH)-NH-C(NH)-NH}\}_n$$

in der n eine Zahl von mindestens 2, insbesondere von 4 bis 6 ist, einschließlich der wasserlöslichen, pharmazeutisch verträglichen Salze derselben, gebildeten Gruppe ausgewählt sind.

Als pharmazeutisch verträgliche Salze der erfindungsgemäß einzusetzenden Bis- bzw. Oligobisguanide eignen sich insbesondere die Hydrochloride, Acetate oder Gluconate; die Verwendung von Chlorhexidin-gluconat ist besonders bevorzugt.

Als Alkohole sind prinzipiell sämtliche Verbindungen geeignet, die in der Pharmazie eingesetzt werden, vorzugsweise solche mit 2 bis 8 Kohlenstoffatomen. Besonders bevorzugt sind Ethanol, n-Propanol und i-Propanol, die einzeln oder in Mischung, gegebenenfalls auch zusammen mit Benzylalkohol, verwendet werden können.

Als weitere Wirkstoffkomponente enthält die erfindungsgemäß zu verwendende Zusammensetzung Wasserstoffperoxid oder in wäßriger Phase Wasserstoffperoxid freisetzende Verbindungen wie Peressigsäure.

Als weitere wesentliche Wirkstoffkomponente enthalten die erfindungsgemäß zu verwendenden Zusammensetzungen eine Carbonsäure oder mehrere. Geeignete Vertreter sind pharmazeutisch verwendbare Carbonsäuren wie Ameisensäure, Essigsäure, Propionsäure, Fumarsäure, Milchsäure, Weinsäure, 9-Undecylensäure, Sorbinsäure und Benzoesäure, die einzeln oder in Mischung eingesetzt werden können. Die Verwendung von Milchsäure ist bevorzugt.

Weiterhin enthalten die erfindungsgemäß zu verwendenden Zusammensetzungen Wasser in einer Menge entsprechend der jeweiligen Applikationsform. Sie werden bevorzugt als wäßrig-alkoholische Lösungen angewendet, können jedoch auch z.B. als filmbildende Präparate, Salben, Emulsionen oder dergleichen formuliert werden.

Gemäß einer weiteren vorteilhaften Ausführungsform der Erfindung enthält die erfindungsgemäß zu verwendende Zusammensetzung
8 bis 25, insbesondere 12 bis 15 Gew.-% Alkohole,
0,2 bis 0,7, insbesondere 0,3 bis 0,6 Gew.-% Wasserstoffperoxid,
0,1 bis 0,5, insbesondere 0,2 bis 0,4 Gew.-% Carbonsäuren und
0,05 bis 1, insbesondere 0,05 bis 0,5 Gew.-% Bis- bzw. Oligobisguanide
in Form eines pharmazeutisch verträglichen Salzes derselben, weiterhin gegebenenfalls übliche Mengen pharmazeutischer Hilfsstoffe wie Parfümstoffe, Farbstoffe, Filmbildner, Emulgatoren, rheologisch wirksame Stoffe, Salbengrundlagen und dergleichen sowie Wasser (auf 100 Gew.-%).

Als besonders geeignete Emulgatoren haben sich Addukte von 35 Mol Ethylenoxid an hydriertes Rhizinusöl oder auch Addukte von mindestens 30 Mol Ethylenoxid an nicht-hydriertes Rhizinusöl und Laurinsäuremonoglycerinester erwiesen; derartige Emulgatoren sind im Handel erhältlich.

Gegebenenfalls können den erfindungsgemäß zu verwendenden Zusammensetzungen auch noch andere, an sich als Desinfektionsmittel bekannte Verbindungen zugesetzt werden, z.B. quartäre Ammoniumverbindungen, Phenolverbindungen und dergleichen; es hat sich jedoch gezeigt, daß für die wirksame Bekämpfung der Eier von Oxyuren die synergistisch wirkende Kombination von Bis- bzw. Oligobisguaniden, Wasserstoffperoxid, Carbonsäuren und Wasser ausreicht.

Die Erfindung wird im folgenden anhand eines bevorzugten Ausführungsbeispiels näher erläutert.

Beispiel.

Es wurde ein Mittel gegen Eier des Madenwurms Enterobius vermicularis der folgenden Zusammensetzung durch Mischung der Einzelkomponenten hergestellt:
10 Gew.-% Ethanol
0,2 Gew.-% Milchsäure
0,5 Gew.-% Wasserstoffperoxid
0,3 Gew.-% Chlorhexidingluconat
0,1 Gew.-% etherisches Öl
0,1 Gew.-% Anlagerungsprodukt von 40 Mol Ethylenoxid an 1 Mol gehärtetes Rhizinusöl
ad 100 Gew.-% Wasser.

Das etherische Öl war eine Mischung aus den folgenden Komponenten:
91,4 % natürliches Pfefferminzöl mit einem Gehalt von ca. 90 % Menthol
4,0 % Salicylsäurephenylester
3,5 % Anethol
0,6 % Eugenol und
0,5 % Thymol.

Die so erhaltene Zusammensetzung ließ sich in Sprayform oder mittels eines Tupfers auf die mit Madenwurmeiern befallene Analhaut eines 3,5-jährigen Patienten auftragen. Die durch die Eiablage hervorgerufenen, unmittelbar erkennbaren Symptome (Juckreiz) verschwanden nahezu augenblicklich. Nach zweimaliger Behandlung waren auch die in den tieferen Falten der Analhaut befindlichen Wurmeier abgetötet. Nach begleitender Behandlung mit einem üblichen Antihelminthikum bei regelmäßiger (morgendlicher) Behandlung mit der erfindungsgemäß zu verwendenden Zusammensetzung war der Patient innerhalb weniger Tage symptomfrei.

**Patentansprüche**

1. Verwendung von Bis- bzw. Oligobisguanide, Alkohole, Wasserstoffperoxid bzw. Wasserstoffperoxid freisetzende Verbindungen, Carbonsäuren und Wasser enthaltenden Zusammensetzungen zur Herstellung eines Arzneimittels zur äußerlichen Anwendung gegen Eier des Madenwurms Enterobius vermicularis auf der Haut.

2. Verwendung nach Anspruch 1, dadurch gekennzeichnet, daß die Bis- bzw. Oligobisguanide aus der von
1,2-Bis-($N^5$-p-chlorphenyl-$N^1$-biguanido)-ethan,
1,2-Bis-($N^5$-p-nitrophenyl-$N^1$-biguanido)-ethan,
1,2-Bis-($N^5$-p-hydroxyphenyl-$N^1$-biguanido)-ethan,
1,2-Bis-($N^5$-p-chlorbenzyl-$N^1$-biguanido)-ethan,
1,2-Bis-($N^5$-p-bromphenyl-$N^5$-hexyl-$N^1$-biguanido)-ethan,
1,2-Bis-($N^5$-p-chlorphenyl-$N^5$-2-ethylphenyl-$N^1$-biguanido)-ethan,
1,2-Bis-($N^5$-p-chlorphenyl-$N^1$-ethyl-$N^1$-biguanido)-ethan,
1,2-Bis-($N^5$-p-methoxyphenyl-$N^1$-biguanido)-ethan,
1,2-Bis-($N^5$-p-methylphenyl-$N^1$-biguanido)-ethan,
1,2-Bis-($N^5$-3,5-dimethylphenyl-$N^1$-biguanido)-ethan,
1,2-Bis-($N^5$-2,6-dichlorphenyl-$N^1$-biguanido)-ethan,
1,2-Bis-($N^5$-2,6-dimethylphenyl-$N^1$-biguanido)-ethan,
1,4-Bis-($N^5$-p-chlorphenyl-$N^1$-biguanido)-butan,
Bis-($N^5$-p-chlorphenyl-$N^1$-biguanido)-methan,

1,3-Bis-($N^5$-p-chlorphenyl-$N^1$-biguanido)-propan
1,6-Bis-($N^5$-p-chlorphenyl-$N^1$-biguanido)-hexan
und Oligobisguaniden der allgemeinen Formel

$[(CH_2)_6\text{-NH-C(NH)-NH-C(NH)-NH}]_n$

in der n eine Zahl von mindestens 2, insbesondere von 4 bis 6 ist, einschließlich wasserlöslichen, pharmazeutisch verträglichen Salzen derselben, gebildeten Gruppe ausgewählt sind.

**3.** Verwendung nach Anspruch 1 oder 2, dadurch gekennzeichnet, daß in der Zusammensetzung als Bisguanid 1,6-Bis-($N^5$-p--chlorphenyl-$N^1$-biguanido)-hexan in Form eines pharmazeutisch verträglichen Salzes enthalten ist.

**4.** Verwendung nach einem der Ansprüche 1 bis 3, dadurch gekennzeichnet, daß in der Zusammensetzung Ethanol als Alkohol enthalten ist.

**5.** Verwendung nach einem der Ansprüche 1 bis 4, dadurch gekennzeichnet, daß die in der Zusammensetzung enthaltenen Carbonsäuren aus der von Ameisensäure, Essigsäure, Propionsäure, Fumarsäure, Milchsäure, Weinsäure, 9-Undecylensäure, Sorbinsäure und Benzoesäure gebildeten Gruppe ausgewählt sind.

**6.** Verwendung nach einem der Ansprüche 1 bis 5, dadurch gekennzeichnet, daß die in der Zusammensetzung enthaltene Carbonsäure Milchsäure ist.

**7.** Verwendung nach einem der Ansprüche 1 bis 6, dadurch gekennzeichnet, daß in der Zusammensetzung

8 bis 25, insbesondere 12 bis 15 Gew.-% Alkohole,

0,2 bis 0,7, insbesondere 0,3 bis 0,6 Gew.-% Wasserstoffperoxid,

0,1 bis 0,5, insbesondere 0,2 bis 0,4 Gew.-% Carbonsäure und

0,05 bis 1, insbesondere 0,05 bis 0,5 Gew.-% eines pharmazeutisch verträglichen Salzes der Bis- bzw. Oligobisguanide

sowie gegebenenfalls übliche Mengen pharmazeutischer Hilfsstoffe wie Parfümstoffe, Farbstoffe, Filmbildner, rheologisch wirksame Substanzen, Emulgatoren und dergleichen, Rest (auf 100 Gew.-%) Wasser, enthalten sind.

## Claims

**1.** The use of compositions containing bis- or oligobisguanides, alcohols, hydrogen peroxide or compounds which release hydrogen peroxide, carboxylic acids and water for the production of a medicament for external application against eggs of the threadworm Enterobius vermicularis on the skin.

**2.** The use claimed in claim 1, characterized in that the bis- or oligobisguanides are selected from the group consisting of 1,2-bis-($N^5$-p-chlorophenyl-$N^1$-biguanido)-ethane,
1,2-bis-($N^5$-p-nitrophenyl-$N^1$-biguanido)-ethane,
1,2-bis-($N^5$-p-hydroxyphenyl-$N^3$-biguanido)-ethane,
1,2-bis-($N^5$-p-chlorobenzyl-$N^1$-biguanido)-ethane,
1,2-bis-($N^5$-p-bromophenyl-$N^5$-hexyl-$N^1$-biguanido)-ethane,
1,2-bis-($N^5$-p-chlorophenyl-$N^5$-2-ethylphenyl-$N^1$-biguanido)-ethane,
1,2-bis-($N^5$-p-chlorophenyl-$N^1$-ethyl-$N^1$-biguanido)-ethane,
1,2-bis-($N^5$-p-methoxyphenyl-$N^1$-biguanido)-ethane,
1,2-bis-($N^5$-p-methylphenyl-$N^1$-biguanido)-ethane,
1,2-bis-($N^5$-3,5-dimethylphenyl-$N^1$-biguanido)-ethane,
1,2-bis-($N^5$-2,6-dichlorophenyl-$N^1$-biguanido)-ethane,
1,2-bis-($N^5$-2,6-dimethylphenyl-$N^1$-biguanido)-ethane,
1,4-bis-($N^5$-p-chlorophenyl-$N^1$-biguanido)-butane,
bis-($N^5$-p-chlorophenyl-$N^1$-biguanido)-methane,
1,3-bis-($N^5$-p-chlorophenyl-$N^1$-biguanido)-propane,
1,6-bis-($N^5$-p-chlorophenyl-$N^1$-biguanido)-hexane
and oligobisguanides corresponding to the following general formula

$[(CH_2)_6\text{-NH-C(NH)-NH-C(NH)-NH}]_n$

in which
n is a number of at least 2, more especially from 4 to 6, including the water-soluble, pharmaceutically acceptable salts thereof.

**3.** The use claimed in claim 1 or 2, characterized in that the composition contains 1,6-bis-($N^5$-p-chlorophenyl-$N^1$-biguanido)-hexane in the form of a pharmaceutically compatible salt as the bisguanide.

**4.** The use claimed in any of claims 1 to 3, characterized in that the composition contains ethanol as the alcohol.

**5.** The use claimed in any of claims 1 to 4, characterized in that the carboxylic acids present in the composition are selected from the group consisting of formic acid, acetic acid, propionic acid, fumaric acid, lactic acid, tartaric acid, 9-undecylenic acid, sorbic acid and benzoic acid.

**6.** The use claimed in any of claims 1 to 5, characterized in that the carboxylic acid in the composition is lactic acid.

**7.** The use claimed in any of claims 1 to 6, characterized in that the composition contains
8 to 25 and more especially 12 to 15% by weight alcohols,
0.2 to 0.7 and more especially 0.3 to 0.6% by weight hydrogen peroxide,
0.1 to 0.5 and more especially 0.2 to 0.4% by weight carboxylic acid and
0.05 to 1 and more especially 0.05 to 0 5% by weight of a pharmaceutically acceptable salt of the bis- or oligobisguanides
and, optionally, typical quantities of pharmaceutical auxiliaries, such as perfumes, dyes, film-forming agents, rheologically active substances, emulsifiers and the like, and water (to 100% by weight).

## Revendications

**1.** Utilisation de compositions contenant les bis- ou oligo-biguanides, des alcools, du peroxyde d'hydrogène ou des composés dégageant du peroxyde d,hydrogène, des acides carboxyliques et de l'eau, pour la préparation d'un médicament pour traitement externe sur la peau contre les oeufs d'Enterobius vermicularis.

**2.** Utilisation selon la revendication 1, caractérisée en ce que les bis- ou oligo-biguanides sont choisis dans le groupe suivant :

1,2-bis-($N^5$-p-chlorophényl-$N^1$-biguanido)-éthane,
1,2-bis-($N^5$-p-nitrophényl-$N^1$-biguanido)-éthane,
1,2-bis-($N^5$-p-hydroxyphényl-$N^1$-biguanido)-éthane,
1,2-bis-($N^5$-p-chlorobenzyl-$N^1$-biguanido)-éthane,
1,2-bis-($N^5$-p-bromophényl-$N^5$-hexyl-$N^1$-biguanido)-éthane,
1,2-bis-($N^5$-p-chlorophényl-$N^5$-2-éthylphényl-$N^1$-biguanido-éthane,
1,2-bis-($N^5$(p-chlorophényl-$N^1$-éthyl-$N^1$-biguanido)-éthane,
1,2-bis-($N^5$-p-méthoxyphényl-$N^1$-biguanido)-éthane,
1,2-bis-($N^5$-p-méthylphényl-$N^1$-biguanido)-éthane,
1,2-bis-($N^5$-3,5-diméthylphényl-$N^1$-biguanido)-éthane,
1,2-bis-($N^5$-2,6-dichlorophényl-$N^1$-biguanido)-éthane,
1,2-bis-($N^5$-2,6-diméthylphényl-$N^1$-biguanido)-éthane,
1,4-bis-($N^5$-p-chlorophényl-$N^1$-biguanido)-butane,
Bis-($N^5$-p-chlorophényl-$N^1$-biguanido)-méthane,
1,3-bis-($N^5$-p-chlorophényl-$N^1$-biguanido)-propane
1,6-bis-($N^5$-p-chlorophényl-$N^1$-biguanido)-hexane

ainsi que les oligo-biguanides selon la formule générale

$$\{(CH_2)_6\text{-NH-C(NH)-NH-C(NH)-NH}\}_n$$

dans laquelle n est un nombre au moins égal à 2 et compris en particulier entre 4 et 6, y compris les sels solubles dans l'eau et pharmaceutiquement acceptables de ces composés.

**3.** Utilisation selon la revendication 1 ou 2, caractérisée en ce que le dérivé de biguanide 1,6-bis-($N^5$-p-chloropnényl-$N^1$-biguanido)-hexane est présent dans la composition sous forme d'un sel pharmaceutiquement acceptable.

**4.** Utilisation selon l'une des revendications 1 à 3, caractérisée en ce que l'alcool contenu dans la composition est de l'éthanol.

**5.** Utilisation selon l'une des revendications 1 à 4, caractérisée en ce que les acides carboxyliques contenus dans la composition sont choisis dans le groupe formé de l'acide formique, de l'acide acétique, de l'acide propionique, de l'acide fumarique, de l'acide lactique, de l'acide tartrique, de l'acide 9-undécylénique, de l'acide sorbique et de l'acide benzoïque.

**6.** Utilisation selon l'une des revendications 1 à 5, caractérisée en ce que l'acide carboxylique contenu dans la composition est de l'acide lactique.

**7.** Utilisation selon l'une des revendications 1 à 6, carcatérisée en ce la composition contient

8 à 25, et plus particulièrement 12 à 15% en poids d'alcools,

0,2 à 0,7, et plus particulièrement 0,3 à 0,6% en poids de peroxyde d'hydrogène,

0,1 à 0,5, et plus particulièrement 0,2 à 0,4% en poids d'acides carboxyliques, et

0,05 à 1, et en particulier 0,05 à 0,5% en poids d'un sel pharmaceutiquement acceptable de bis-ou oligobiguanide

ainsi que, éventuellement, des quantités usuelles d'adjuvants pharmaceutiques tels que parfums, colorants, agents filmogènes, émulsifiants, substances à effet rhéologique et produits analogues, le reste à 100% en poids étant formé d'eau.